# EUROPEAN PATENT APPLICATION

(11) **EP 0 957 090 A1**
(43) Date of publication of application: **17.11.1999**
(21) Application number: 99303723.3
(22) Date of filing: 12.05.1999
(51) Int. Cl.: C07D 205/08

(54) **Method for producing 3,3,4,4-tetramethyl-2-azetidinone**

(30) Priority: 13.05.1998 JP 13036798
(71) Applicant: Sumitomo Chemical Company, Limited, Chuo-ku Osaka 541-8550 (JP)
(72) Inventor: Haga, Toru, Niihama-shi, Ehime (JP); Imi, Katsuharu, Niihama-shi, Ehime (JP); Ogawa, Takeshi, Narashino-shi, Chiba (JP)
(74) Representative: Cresswell, Thomas Anthony

(57) **Abstract**

The present invention provides a method for producing 3,3,4,4-tetramethyl-2-azetidinone comprising removing a chlorosulfonyl group from 1-chlorosulfonyl-3,3,4,4-tetramethyl-2-azetidinone using an alkali compound in the presence of a phase transfer catalyst, which has excellent operability and high yield and provides easy treatment of the waste liquid.

## Description

The present invention relates to a method for producing 3,3,4,4-tetramethyl-2-azetidinone useful as an intermediate of medicine, agricultural chemical, additive and the like.

For producing 3,3,4,4-tetramethyl-2-azetidonine, there is a method for removing a chlorosulfonyl group of 1-chlorosulfonyl-3,3,4,4-tetramethyl-2-azetidinone, and as such methods, there are known a method (1) in which the removal of a chlorosulfonyl group is conducted via reduction using benzenethiol and pyridine(Journal of Organic Chemistry, vol.33, p.344-8, 1968 and ibid. vol.36, p.2841, 1971), a method(2) in which the removal of a chlorosulfonyl group is conducted via hydrolysis by adding an aqueous sodium hydroxide solution in acetone(Journal of Organic Chemistry, vol.36, p.2841, 1971), and a method(3) in which the removal of a chlorosulfonyl group is conducted via reduction using sodium sulfite and potassium hydroxide(Journal of Organic Chemistry, vol.35, p.2043, 1970).

However, the method(1) for removing a chlorosulfonyl group using benzenethiol and pyridine has problems that odoriferous compounds such as benzenethiol and pyridine are used in large amounts(more than stoichiometric amounts), a diphenyl disulfide in stoichiometric amount is produced as a by-product and a separation of it is necessary, the reaction is required to be conducted at lower temperature, the yield is low, the treatment of the waste liquid is not easy, and the like. The method(2) for removing a chlorosulfonyl group by adding an aqueous sodium hydroxide solution in acetone has problems that extraction efficiency is poor because acetone is mixed into the aqueous layer in conducting extraction, yield is low, the treatment of the waste liquid is not easy, and the like. Further, the method(3) for removing a chlorosulfonyl group using sodium sulfite and potassium hydroxide has problems that the reaction reagents have to be supplied with maintaining pH of the reaction mixture at somewhat alkaline side, the waste liquid containing a large amount of sodium sulfite is produced, and the like, while the yield is relatively higher as compared with the above-described another methods.

Thus, any of the conventional methods has various problems in the points of operability, yield and waste water, and is not suitable for industrial production.

The present inventors have intensively studied a method for producing 3,3,4,4-tetramethyl-2-azetidinone for solving the problems of the conventional methods, and as a result, found that the problems of the conventional methods can be solved by removing a chlorosulfonyl group from 1-chlorosulfonyl-3,3,4,4-tetramethyl-2-azetidinone as a raw material using an alkali compound in the presence of a phase transfer catalyst, completing the present invention.

An object of the present invention is to provide a method for producing 3,3,4,4-tetramethyl-2-azetidinone which has excellent operability, high yield and provides easy treatment of the waste liquid.

Namely, the present invention provides a method for producing 3,3,4,4-tetramethyl-2-azetidinone comprising removing a chlorosulfonyl group from 1-chlorosulfonyl-3,3,4,4-tetramethyl-2-azetidinone using an alkali compound in the presence of a phase transfer catalyst.

The present invention will be described further in detail below.

The method for producing l-chlorosulfonyl-3,3,4,4-tetramethyl-2-azetidinone used as raw material in the present invention, and kind and content of impurities in raw material are not particularly restricted. As a method for producing this raw material, for example, there is a method in which 2,3-dimethyl-2-butene and chlorosulfonyl isocyanate are subjected to cycloaddition in an organic solvent. When 1-chlorosulfonyl-3,3,4,4-tetramethyl-2-azetidinone is produced in this method, the reaction mixture contained 1-chlorosulfonyl-3,3,4,4-tetramethyl-2-azetidinone may be used as it is in the present invention, or the purified 1-chlorosulfonyl-3,3,4,4-tetramethyl-2-azetidinone obtained by crystallization of the reaction mixture and the like may be used in the present invention.

As the phase transfer catalyst used in the present invention, quaternary ammonium salts such as benzyltriethylammonium chloride, tetrabutylammonium bromide and the like, quaternary phosphonium salts such as tetrabutylphosphonium bromide and the like, crown ethers such as 18-crown-6,15-crown-5 and the like, cryptands, tris[2-(2-methoxyethoxy)ethyl]amine, and the like are listed, and at least one of them is used. Among them, benzyltriethylammonium chloride is preferably used.

In the present invention, an alkali compound is added in the reaction system. The alkali compound used is not particularly restricted, and sodium hydroxide, potassium hydroxide, lithium hydroxide, barium hydroxide and the like are usually listed.

The reaction of the present invention is conducted in a solution or suspension, and the alkali compound is preferably dissolved in water for use in the reaction.

The amount of the alkali compound used may be stoichiometric amount (3 mol per 1 mol of 1-chlorosulfonyl-3,3,4,4-tetramethyl-2-azetidinone) or more, and preferably about 3.05 to about 5.0 mol per 1 mol of 1-chlorosulfonyl-3,3,4,4-tetramethyl-2-azetidinone.

If impurities consuming an alkali compound are contained in the raw material, the consuming amount should be took into consideration.

The amount of the phase transfer catalyst used is about 0.001 to about 0.05 part by weight, preferably about 0.002 to about 0.02 part by weight per 1 part by weight of 1-chlorosulfonyl-3,3,4,4-tetramethyl-2-azetidinone. When the amount of the catalyst is less than the above-described amount, the progress of the reaction is not sufficient, and on the other, when more than the above-described amount, the production cost increases though the reaction progresses.

The reaction temperature is selected in the range from about 40 to about 100°C, preferably in the range from about 45 to about 60°C. The reaction time varies depending on the amount of the reaction materials, reaction temperature and the like, and selected in the range from about 0.5 to about 48 hours.

A method for conducting the reaction is not particularly restricted, and for example, it may be permissible that a solution prepared by dissolving or suspending an alkali compound and a phase transfer catalyst in water is heated, and to this solution is added a solution prepared by dissolving or suspending 1-chlorosulfonyl-3,3,4,4-tetramethyl-2-azetidinone in an organic solvent, that a solution prepared by dissolving or suspending 1-chlorosulfonyl-3,3,4,4-tetramethyl-2-azetidinone and a phase transfer catalyst in organic solvent is heated, and to this solution is added a solution prepared by dissolving or suspending an alkali compound in water, or that a solution prepared by mixing an alkali compound, phase transfer catalyst, water and organic solvent is heated, and to this solution is added 1-chlorosulfonyl-3,3,4,4-tetramethyl-2-azetidinone.

The method in which a solution prepared by dissolving an alkali compound and a phase transfer catalyst in water is heated, and to this solution is added a solution prepared by dissolving 1-chlorosulfonyl-3,3,4,4-tetramethyl-2-azetidinone in an organic solvent is preferably used in view of reactivity and operability.

The treatment of the reaction mixture after completion of the reaction is not particularly restricted, and for example, it is also permissible that the organic layer is separated from reaction mixture as it is, or from solution added optionally water, an organic solvent and the like to the reaction mixture before separation, the aqueous layer is extracted with an organic solvent if necessary, and the organic layer separated is concentrated.

As the organic solvent used in dissolving raw materials and/or used in separation, there are usually used aromatic hydrocarbons such as benzene, toluene, xylene, ethylbenzene, mesitylene and the like, and ethers such as diethyl ether, diisopropyl ether, t-butyl methyl ether and the like in view of no reactivity with raw materials and product, separating property from water, and the like. Among them, toluene is preferably used in view of excellent operability, ability for dissolving raw materials , ability for extracting the product, and the like.

Thus obtained 3,3,4,4-tetramethyl-2-azetidinone is further purified by a means such as re-crystallization, chromatography and the like, if necessary.

The waste liquid generated in the method of the present invention contains only contains a small amount of raw materials, product and solvent in addition to excess alkali compound and alkali salts of hydrogen chloride and sulfuric acid generated by removal of chlorosulfonyl group using alkali compound, then, the treatment thereof is easier as compared with the conventional methods.

The present invention has more excellent operability, can produce 3,3,4,4-tetramethyl-2-azetidinone from 1-chlorosulfonyl-3,3,4,4-tetramethyl-2-azetidinone in higher yield, and provides easier treatment of the waste liquid as compared with the conventional methods, and industrial availability thereof is extremely high.

### EXAMPLE

The following examples illustrate the present invention further in detail below, but do not limit the scope thereof.

### Example 1

Into a 2-liter round bottom flask equipped with a thermometer, dropping funnel, condenser and mechanical stirred was charged 375.0 g of a 25% aqueous sodium hydroxide solution(3.53 mol per 1 mol of 1-chlorosulfonyl-3,3,4,4-tetramethyl-2-azetidinone), and to this solution was added 1.50 g of benzyltriethylammonium chloride (0.01 part by weight per 1 part by weight of 1-chlorosulfonyl-3,3,4,4-tetramethyl-2-azetidinone) at room temperature, the resulted mixture was heated to 50°C, and to this mixture was added dropwise a solution prepared by dissolving 150.0 g (0.6646 mol) of 1-chlorosulfonyl-3,3,4,4-tetramethyl-2-azetidinone in 600 g of toluene, through the dropping funnel over 1 hour. The mixture was kept at 50°C for 1 hour, then, 100.60 g of water was added, the organic layer was separated. The aqueous layer was extracted with 300 g of toluene. The organic layer was joined, toluene was distilled off under reduced pressure at a temperature of 40°C or lower, and the residue was dried under reduced pressure to obtain 82.81 g (yield: 98%) of 3,3,4,4-tetramethyl-2-azetidine. The resulted 3,3,4,4-tetramethyl-2-azetidine was analyzed by gas chromatography to find that the content was 99.8% in terms of area percentage.

### Examples 2, 3

Reactions were conducted in the same manner as in Example 1 except that the amount used of sodium hydroxide was changed as shown in mole ratio in Table 1. The yield of the resulted 3,3,4,4-tetramethyl-2-azetidinone is shown in Table 1.

**[Table 1]**

| | Mole ratio of sodium hydroxide to 1-chlorosulfonyl-3,3,4,4-tetramethyl-2-azetidinone | Yield (%) of 3,3,4,4-tetramethyl-2-azetidinone |
|---|---|---|
| Example 2 | 5.0 | 97 |
| Example 3 | 3.1 | 98 |

### Example 4, Comparative Example 1

Reactions were conducted in the same manner as in Example 1 except that the amount used of benzyltriethylammonium chloride was changed as shown in ratio by weight in Table 2. The yield of the resulted 3,3,4,4-tetramethyl-2-azetidinone is shown in Table 2.

**[Table 2]**

| | Ratio by weight of benzyltrimethylammonium chloride to 1-chlorosulfonyl-3,3,4,4-tetramethyl-2-azetidinone | Yield (%) of 3,3,4,4-tetramethyl-2-azetidinone |
|---|---|---|
| Example 4 | 0.0025 | 98 |
| Comparative example 1 | 0 | * |

| | | |
|---|---|---|
| * in Table 2 has the following meaning: | | |

An unreacted 1-chlorosulfonyl-3,3,4,4-tetramethyl-2-azetidinone remained in a ratio of 66% based on the amount used in the reaction mixture after the temperature was kept for 4 hours.

### Example 5

Into the same apparatus as in Example 1 was charged 248 g (1.55 mol) of a 25% aqueous sodium hydroxide solution, and to this solution was added 0.5 g of benzyltriethylammonium chloride, the resulted mixture was heated to 50°C, and to this mixture was added dropwise a solution prepared by diluting 200 g of a reaction mixture obtained by reaction of chlorosulfonyl isocyanate with 2,3-dimethyl-2-butene in a toluene solvent(the content of 1-chlorosulfonyl-3,3,4,4-tetramethyl-2-azetidinone in the reaction mixture was 78 g(0.346 mol)) with 300 g of toluene, through the dropping funnel over 1 hour. The mixture was kept at 50°C for 1 hour, then, 50 g of toluene and 75 g of water were added, the organic layer was separated. The aqueous layer was extracted with 200 g of toluene. The organic layer was joined, toluene was distilled off under reduced pressure to obtain 48 g of a crude crystal(the content of 3,3,4,4-tetramethyl-2-azetidinone in the crude crystal was 40 g(0.314 mol), yield is 91%). 48 g of this crude crystal was dissolved in 48 g of toluene at 70°C, then the solution was cooled to 5°C for re-crystallization to obtain 35 g of a crystal (purity 99% or more) of 3,3,4,4-tetramethyl-2-azetidinone.

### Comparative Example 2

2.4 g of sodium sulfite was dissolved in 24 g of water, the solution was heated to 40°C, and to this solution was added dropwise a solution prepared by dissolving 20.0 g (0.08861 mol) of 1-chlorosulfonyl-3,3,4,4-tetramethyl-2-azetidinone in 80 g of toluene, over 1 hour. During this procedure, a 23% aqueous sodium hydroxide solution was added dropwise so that pH in the reaction mixture was maintained at from 7 to 8. The mixture was kept at 40°C for 1.5 hours, the organic layer was separated, then, the aqueous layer was extracted with 40 g of toluene. The organic layer was joined, the mixture was washed with 40 g of water, then, concentrated the organic solution and dried to obtain 8.72 g (purity 99.8%, yield 77%) of 3,3,4,4-tetramethyl-2-azetidinone. The aqueous layers in the extraction and washing contained 3,3,4,4-tetramethyl-2-azetidinone in an yield of 14% in total.

## Claims

1. A method for producing 3,3,4,4-tetramethyl-2-azetidinone comprising removing a chlorosulfonyl group from 1-chlorosulfonyl-3,3,4,4-tetramethyl-2-azetidinone using an alkali compound in the presence of a phase transfer catalyst.

2. A method according to claim 1, wherein the phase transfer catalyst is at least one compound selected from quaternary ammonium salts, quaternary phosphonium salts, crown ethers, cryptands and tris[2-(2-methoxyethoxy)ethyl]amine.

3. A method according to claim 1 or 2, wherein the phase transfer catalyst is benzyltriethylammonium chloride.

4. A method according to any one of the preceding claims, wherein the alkali compound is sodium hydroxide.

5. A method according to any one of the preceding claims, wherein a reaction mixture obtained by cycloaddition of 2,3-dimethyl-1-butene and chlorosulfonyl isocyanate is used as it is as 1-chlorosulfonyl-3,3,4,4-tetramethyl-2-azetidinone.

6. A method according to any one of the preceding claims, wherein the method is conducted in the presence of an organic solvent.

7. A method according to claim 6, wherein the organic solvent is toluene.

8. A method according to any one of the preceding claims, wherein the amount of the phase transfer catalyst is from 0.001 to 0.05 parts by weight per 1 part by weight of 1-chlorosulfonyl-3,3,4,4-tetramethyl-2-azetidinone.

9. A method according to any one of the preceding claims, wherein the amount of the alkali compound is from 3.05 to 5.0 mol per 1 mol of 1-chlorosulfonyl-3,3,4,4-tetramethyl-2-azetidinone.
